# EUROPEAN PATENT APPLICATION

(11) **EP 2 724 721 A1**
(43) Date of publication of application: **30.04.2014**
(21) Application number: 12007369.7
(22) Date of filing: 26.10.2012
(51) Int. Cl.: A61K 31/138, A61K 31/56, A61K 31/565, A61P 25/00

(54) **Composition for use in the treatment of Angelman syndrome and/or autism spectrum disorder, the use of such composition and a method for manufacturing a medicament for the treatment of Angelman syndrome and/or autism spectrum disorder**

(71) Applicant: Matentzoglu, Konstantin, 88718 Daisendorf (DE)
(72) Inventor: Dr. Konstantin MATENTZOGLU, 88718 Daisendorf (DE)
(74) Representative: Kunz, Herbert

(57) **Abstract**

The invention is related to a composition for use in the treatment of Angelman syndrome and/or autism spectrum disorder, wherein the composition comprises a substance that restores deregulated steroid hormone signaling and /or restores the expression levels or neuronal activity of E3 ubiquitin-protein ligase E6APmodulates the steroid hormone signaling in order to compensate for deregulation. Furthermore, the invention is directed to the use of such composition and a method for manufacturing a medicament intended for therapeutic application in the treatment of Angelman syndrome and/or autism spectrum disorder.

## Description

The present invention relates to a composition for use in the treatment of Angelman syndrome and/or autism and/or autism spectrum disorders and methods for detecting mechanistic pathways thereof. Furthermore, the invention is directed to the use of such composition and a method for manufacturing a medicament intended for therapeutic application in the treatment of Angelman syndrome and/or autism and/or autism spectrum disorders.

### State of the art

### E6AP

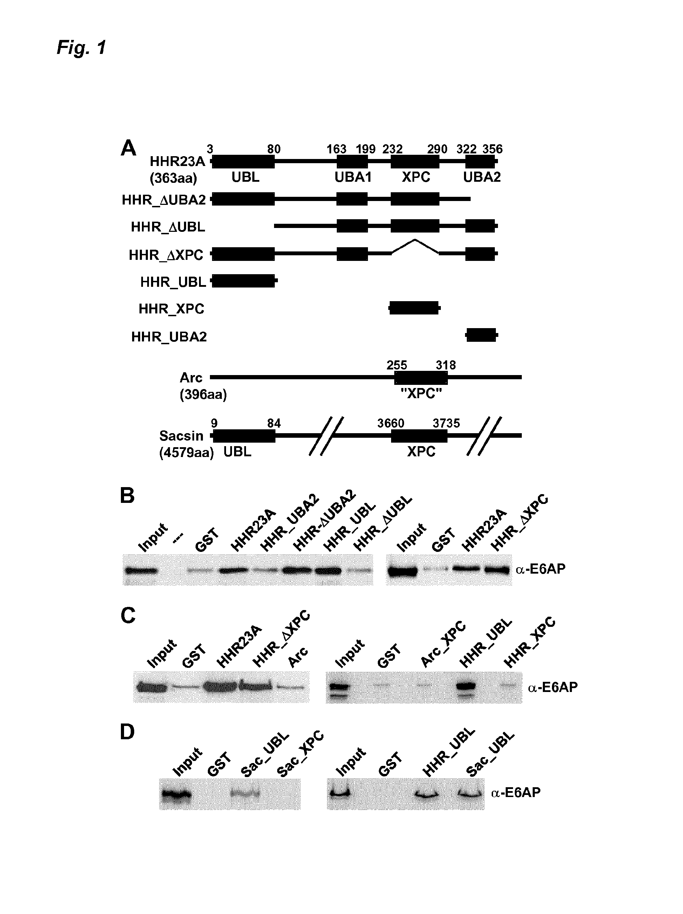

The ubiquitin ligase E6AP (E6-associated protein) represents the founding member of the HECT (homologous to E6AP C terminus) family of E3 ubiquitin ligases [1-3]. E6AP is encoded by the UBE3A gene located on chromosome 15q11-13 [4-7]. The UBE3A gene encodes three protein isoforms generated by differential splicing [8]. These isoforms differ at their very N-termini and consist of 852 amino acids (isoform 1), 872 amino acids (isoform 3), and 875 amino acids (isoform 2), respectively (in the following, numbering of amino acid residues is according to isoform 1). It is presently unknown if the isoforms have different properties (e.g. towards putative target proteins) and/or show tissue-specific expression. Intriguingly, deregulation of the E3 activity of E6AP has been associated with two distinct human diseases [6,reviewed in 9,10] and, thus, E6AP may currently represent the most prominent example for the notion that inappropriate modulation of the activity of components of the ubiquitin-conjugation system contributes to the development of human disease: Unscheduled activation of E6AP contributes to cervical carcinogenesis ("gain of function"), while inactivation results in a neurodevelopmental disorder, the Angelman syndrome (AS) ("loss of function") (Fig. 1 B). Taken together, these facts necessitate the thorough elucidation of cellular pathways involving E6AP.

### "Gain of function": E6AP and cervical cancer

It is generally accepted that infection with certain types of human papillomaviruses (HPVs) represents the most significant risk factor for the development of cervical cancer (for review, see [9]). Approximately 30 HPV types have been associated with lesions of the anogenital tract and these HPVs can be roughly classified into "low risk" and "high risk" types based on their association with clinical lesions. While low risk HPVs are generally associated with benign lesions including condyloma accuminata, high risk HPVs have been etiologically associated with malignant lesions of the anogenital tract, most notably cervical cancer. High risk HPVs encode two major oncoproteins termed E6 and E7 and the respective genes are the only viral genes that are generally retained and expressed in cervical cancer tissues. Furthermore, a number of studies have shown that continuous expression of both E6 and E7 is required for the viability of HPV-positive cancer cells (for reviews, see [9-11]). Since cervical cancer represents the second most common cancer in women, it has been, and still is, of considerable interest to elucidate and characterize the oncogenic functions of E6 and E7.

E6AP was originally isolated as a protein that binds to the E6 oncoprotein of human papillomaviruses (HPVs) associated with cervical cancer and, in complex with E6, targets the tumor suppressor p53 for ubiquitination and proteasome-mediated degradation [1,12]. The region comprising amino acids 378-401 of E6AP serves as binding site for HPV E6 proteins [13] (Fig. 1A). This region is necessary and sufficient for the E6/E6AP interaction and even subtle changes within this region interfere with E6 binding [14]. However, p53 is not the only target of the E6/E6AP complex. Besides p53, known substrates of the E6/E6AP complex include several PDZ domain containing proteins (e.g. hDlg, Scribble), E6TP1, and NFX1-91, a transcriptional repressor of the gene encoding hTERT, the rate-limiting catalytic subunit of telomerase [15-20] (for reviews, see [10,11]). The notion that the ability of E6 to utilize E6AP to target p53 and other cellular proteins for degradation contributes to its oncogenic functions is supported by several observations: (i) Transgenic mouse models indicate that both ablation of p53 activity and the interaction with PDZ domain proteins are critical for HPV-induced tumorigenesis [21,22]; (ii) In contrast to many other tumor types, the p53 gene is very rarely mutated in cervical carcinomas [9,10]. Thus, E6/E6AP-induced degradation of p53 can be considered as functionally equivalent to inactivation of p53 by mutation of the p53 gene, although the situation in HPV-positive cancers may be somewhat more complicated (for detailed discussion of this issue, see [10]); and (iii) Interference with E6AP expression by antisense RNA-based approaches or by RNA interference results in accumulation of p53 and activation of its transcriptional and growth-suppressive properties in HPV-positive but not in HPV-negative cells (indicating that E6AP plays no or only a minor role in p53 degradation in the absence of E6) [23-26].

Finally, it should be noted that E6AP may not only be utilized by E6 but, in addition, may represent a direct target for E6, since binding of E6 targets E6AP for ubiquitination and degradation [27]. Although the physiological relevance of this finding is not yet understood, it can be speculated that an E6-induced decrease of intracellular E6AP levels should have profound effects on the stability of E6-independent substrates of E6AP.

### "Loss of function": E6AP and Angelman syndrome

AS was first described in 1965 by the pediatrician Harry Angelman [28,29]. AS is a genetic disorder with an incidence of approx. 1 in 10,000 to 1 in 40,000 and is characterized by mental retardation, movement or balance disorder, characteristic abnormal behaviors, and severe limitations in speech and language. Development of AS has been linked to chromosome 15q11-13, which is known as the Prader-Willi/Angelman syndrome region and, as indicated above, contains the UBE3A gene. The Prader-Willi/Angelman syndrome region comprises approx. 4 megabases and contains a bipartite imprinting center and, thus, maternally and paternally imprinted genes [30]. The Prader-Willi syndrome (PWS) and the Angelman syndrome (AS) represent two clinically distinct neurodevelopmental disorders with PWS resulting from paternal genetic deficiency and AS from maternal genetic deficiency [5]. Importantly, the paternal UBE3A allele is imprinted in certain brain areas (Purkinje neurons and a subset of hippocampal neurons), but expressed from both alleles in all other tissues throughout the body [31,32]. Furthermore, all of the genetic abnormalities associated with AS affect expression of the maternal UBE3A gene and/or the E3 activity of E6AP. Genetic abnormalities include deletion of the 15q11-13 region of the maternal chromosome (accounting for approx. 70 percent of the AS cases), uniparental paternal disomy, defects in imprinting, and single point mutations in the UBE3A gene [5,29,33]. However, why loss of E6AP activity results in AS development is not understood, mainly because the cellular pathways and processes that are controlled by E6AP have not yet been identified. In this context, it should be noted that cell culture studies indicate that E6AP positively affects the activity of nuclear hormone receptors (estrogen, androgen, progesterone receptors) and that this property of E6AP is not related to its E3 function [34]. The effect on hormone receptors appears to be mediated via two spatially separated amino acid motifs (one within the E6 binding site and the other within the HECT domain) of E6AP that match the consensus sequence of a nuclear hormone receptor interaction motif (LxxLL) [34,35]. However, the relevance of the ability of E6AP to affect nuclear hormone receptor activity for the development of AS is unclear (e.g. this property is not affected in those E6AP mutants that are derived from AS patients with point mutations in the UBE3A gene).

Finally, deregulation of E6AP expression/activity has also been discussed to contribute to autism [e.g. 36,37]. However, the available data are purely descriptive and, apart from altered expression profiles, do not provide any evidence for a direct contribution of deregulated E6AP activity to the development of autism.

### E6AP in mice

Two Ube3a knockout mouse models (Ube3a^{tm1Alb}, Ube3a^{tm1Jwf}) are currently available [38,39]. The respective mice display a number of features reminiscent of AS patients including motor dysfunction, mental retardation, seizures, and sleep disturbances [38-40]. Remarkably, Ube3a transgenic mice develop these AS like features by disruption of only the maternal allele, thus reflecting the situation in AS patients. Indeed, similar to the human UBE3A gene, the mouse Ube3a gene is biallelically expressed in all somatic cells with the exception of Purkinje cells (cerebellum), hippocampal neurons, and mitral cells of the olfactory bulb, in which the paternally derived Ube3a gene is silenced [41].

The abovementioned data indicate that Ube3a knockout mice provide invaluable tools for the elucidation of the molecular mechanisms underlying the neurodevelopmental defects of AS patients. This notion is underlined by experiments studying ataxia, one of the diagnostic criteria of AS. In general, ataxia can result from various dysfunctions of the cerebellar cortex. In Ube3a knockout mice, the cortical dysfunction may arise from fast oscillation (approximately 160 Hz) in the cerebellar cortex, which is sustained by an abnormally increased firing rate and rhythmicity of (E6AP-deficient) Purkinje cells [42]. This oscillation is inhibited by sensory stimulation or by gap junction and GABA(A) receptor blockers, respectively. Of note, a similar oscillation was previously found in mice lacking calcium-binding proteins that also present ataxia, but never in wild-type mice [43]. Thus, it has been proposed that fast oscillation in the cerebellar cortex is implicated in the cerebellar symptomatology of AS and thus, inhibition of this oscillation might alleviate symptoms of ataxia in AS patients [42].

### "E6APomics"

To understand why loss of the E3 activity of E6AP results in the development of AS, it is essential to identify the cellular pathways that involve E6AP. An obvious possibility to do so is the identification and characterization of proteins that interact with E6AP and modulate its function and/or serve as ubiquitination substrates of E6AP. Several E6-independent substrates of E6AP have been reported, including HHR23A and HHR23B (the human homologs of *S*. *cerevisiae* RAD23), Blk (member of the Src-family of nonreceptor tyrosine kinases), Bak (human pro-apoptotic protein), Mcm7 (which is involved in DNA replication), and AIB1 (coactivator of steroid receptors) [44-48]. However, if deregulated degradation of these proteins is involved in the pathogenesis of AS patients is presently unclear. In addition, in one of the Ube3a knockout mouse models, cytoplasmic levels of p53 have been reported to be significantly increased in postmitotic neurons [38]. However, since this was not observed in the other Ube3a knockout mouse model [39] and since all available data obtained in cell culture systems indicate that, in the absence of HPV E6, E6AP does not play a prominent role in p53 degradation, it seems likely that the observed increase in p53 levels is an indirect rather than a direct effect of loss of E6AP expression.

### E6AP: A therapeutic target?

The data available suggest that the ability of the HPV E6 oncoprotein to target p53 and other cellular proteins for degradation is required for the viability of cell lines derived from cervical cancer patients [26,51]. Thus, the E6/E6AP complex represents a bona fide target for molecular approaches in the treatment of cervical cancer. However, since E6AP is expressed in all human tissues, E6 (since it is a viral protein) rather than E6AP should be the target of choice for potential anticancer strategies.

AS is caused by loss of E6AP expression indicating that proteins acting upstream or downstream of pathways involving E6AP rather than E6AP represent targets for potential therapeutic approaches. However, AS is a neurodevelopmental disorder and it is presently unclear if the neuronal defects caused by loss of E6AP activity can be reverted and, if so, at which stage of neuronal development. As a first step to answer this question, the availability of Ube3A knockout mice should prove helpful. For example, it has been reported that hippocampal slices derived from Ube3A knockout mice show deficits in long-term potentiation [38]. If it is possible to rescue this phenotype by restoration of E6AP expression, this would suggest that it should be possible to devise strategies to improve the conditions of AS patients.
1 Scheffner, M., Huibregtse, J.M., Vierstra, R.D., Howley, P.M. (1993) The HPV-16 E6 and E6AP complex functions as a ubiquitin-protein ligase in the ubiquitylation of p53. Cell 75, 495-505
2 Huibregtse, J.M., Scheffner, M., Beaudenon, S., Howley, P.M. (1995) A family of proteins structurally and functionally related to the E6AP ubiquitin-protein ligase. Proc. Natl. Acad. Sci. 92, 2563-2567.
3 Scheffner, M., Nuber, U., Huibregtse, J.M. (1995) Protein ubiquitination involving an E1-E2-E3 enzyme ubiquitin thioester cascade. Nature 373, 81-83
4 Nakao, M., Sutcliffe, J.S., Durtschi, B., Mutirangura, A., Ledbetter, D.H., Beaudet, A.L. (1994) Imprinting analysis of three genes in the Prader-Willi/Angelman region: SNRPN, E6-associated protein, and PAR-2 (D15S225E). Hum. Mol. Genet. 3(2), 309-15
5 Nicholls, R.D., Knepper, J.L. (2001) Genome organization, function, and imprinting in Prader-Willi and Angelman syndromes. Annu. Rev. Genomics. Hum. Genet. 2, 153-175
6 Kishino, T., Lalande, M., Wagstaff, J. (1997) UBE3A/E6AP mutations cause Angelman syndrome. Nat. Genet. 15, 70-73
7 Matsuura, T., Sutcliffe, J.S., Fang, P., Galjaard, R.J., Jiang, Y.H., Benton, C.S., Rommens, J.M., Beaudet, A.L. (1997) De novo truncating mutations in E6AP ubiquitin-protein ligase gene (UBE3A) in Angelman syndrome. Nat. Genet. 15, 74-77
8 Yamamoto, Y., Huibregtse, J.M., Howley, P.M. (1997) The human E6AP gene (UBE3A) encodes three potential protein isoforms generated by differential splicing. Genomics 41, 263-266
9 zur Hausen H (2002) Papillomaviruses and cancer: from basic studies to clinical application. Nat. Rev. Cancer 2, 342-350
10 Scheffner M, Whitaker, N.J. (2003) Human papillomavirus-induced carcinogenesis and the ubiquitin-proteasome system. Semin. Cancer Biol. 13, 59-67
11 Mantovani, F., Banks, L. (2001) The human papillomavirus E6 protein and its contribution to malignant progression. Oncogene 20, 7874-7887
12 Huibregtse, J.M., Scheffner, M., Howley, P.M. (1993) Cloning and expression of the cDNA for E6AP, a protein that mediates the interaction of the human papillomavirus E6 oncoprotein with p53. Mol. Cell. Biol. 13, 775-784
13 Huibregtse, J.M., Scheffner, M., Howley, P.M. (1993) Localization of the E6AP regions that direct human papillomavirus E6 binding, association with p53, and ubiquitination of associated proteins. Mol. Cell. Biol. 13, 4918-4927
14 Be, X., Hong, Y., Wei, J., Androphy, E.J., Chen, J.J., Baleja, J.D. (2001) Solution structure determination and mutational analysis of the papillomavirus E6 interacting peptide of E6AP. Biochemistry 40, 1293-1299
15 Nakagawa, S., Huibregtse, J.M. (2000) Human scribble (Vartul) is targeted for ubiquitin-mediated degradation by the high-risk papillomavirus E6 proteins and the E6AP ubiquitin-protein ligase. Mol. Cell. Biol. 20, 8244-8253
16 Gewin, L., Myers, H., Kiyono, T., Galloway, D.A. (2004) Identification of a novel telomerase repressor that interacts with the human papillomavirus type-16 E6/E6AP complex. Genes Dev. 18, 2269-2282
17 Töpffer, S., Müller-Schiffmann, A., Matentzoglu, K., Scheffner, M., Steger, G. (2007) Protein tyrosine phosphatase H1 is a target of the E6 oncoprotein of high-risk genital human papillomaviruses. J. Gen. Virol. 88, 2956-65
18 Kuballa, P., Matentzoglu, K., Scheffner, M. (2007) The role of the ubiquitin ligase E6AP in human papillomavirus E6-mediated degradation of PDZ domain-containing proteins. J. Biol. Chem. 5, 65-71
19 Jing, M., Bohl, J., Brimer, N., Kinter, M., Vande Pol, S.B. (2007) Degradation of tyrosine phosphatase PTPN3 (PTPH1) by association with oncogenic human papillomavirus E6 proteins. J. Virol. 81, 2231-9
20 Gao, Q., Kumar, A., Singh, L., Huibregtse, J.M., Beaudenon, S., Srinivasan, S., Wazer, D.E., Band, H., Band, V. (2002) Human papillomavirus E6-induced degradation of E6TP1 is mediated by E6AP ubiquitin ligase. Cancer Res. 62, 3315-21
21 Pan, H., Griep, A.E. (1994) Altered cell cycle regulation in the lens of HPV-16 E6 or E7 transgenic mice: implications for tumor suppressor gene function in development. Genes Dev. 8, 1285-1299
22 Simonson, S.J., Difilippantonio, M.J., Lambert, P.F. (2005) Two distinct activities contribute to human papillomavirus 16 E6's oncogenic potential. Cancer Res. 65, 8266-8273
23 Beer-Romero, P., Glass, S., Rolfe, M. (1997) Antisense targeting of E6AP elevates p53 in HPV-infected cells but not in normal cells. Oncogene 14, 595-602
24 Kim, Y., Cairns, M.J., Marouga, R., Sun, L.Q. (2003) E6AP gene suppression and characterization with in vitro selected hammerhead ribozymes. Cancer Gene Ther. 10, 707-716
25 Kelley, M.L., Keiger, K.E., Lee, C.J., Huibregtse, J.M. (2005) The global transcriptional effects of the human papillomavirus E6 protein in cervical carcinoma cell lines are mediated by the E6AP ubiquitin ligase. J. Virol. 79, 3737-3747
26 Hengstermann, A., D'silva, M.A., Kuballa, P., Butz, K., Hoppe-Seyler, F., Scheffner, M. (2005) Growth suppression induced by downregulation of E6AP expression in human papillomavirus-positive cancer cell lines depends on p53. J. Virol. 79, 9296-92300
27 Kao, W.H., Beaudenon, S.L., Talis, A.L., Huibregtse, J.M., Howley, P.M. (2000) Human papillomavirus type 16 E6 induces self-ubiquitination of the E6AP ubiquitin-protein ligase. J. Virol. 74, 6408-6417
28 Angelman, H. (1965) "Puppet children". A report of three cases. Dev. Med. Child Neurol. 7, 681-688
29 Clayton-Smith, J., Laan, L. (2003) Angelman syndrome: a review of the clinical and genetic aspects. J. Med. Genet. 40, 87-95
30 Amos-Landgraf, J.M., Ji, Y., Gottlieb, W., Depinet, T., Wandstrat, A.E., Cassidy, S.B., Driscoll, D.J., Rogan, P.K., Schwartz, S., Nicholls, R.D. (1999) Chromosome breakage in the Prader-Willi and Angelman syndromes involves recombination between large, transcribed repeats at proximal and distal breakpoints. Am. J. Hum. Genet. 65, 370-386
31 Vu, T.H., Hoffman, A.R. (1997) Imprinting of the Angelman syndrome gene, UBE3A, is restricted to brain. Nat. Genet. 17, 12-13
32 Rougeulle, C., Glatt, H., Lalande, M. (1997) The Angelman syndrome candidate gene, UBE3A/E6AP, is imprinted in brain. Nat. Genet. 17, 14-15
33 Fang, P., Lev-Lehman, E., Tsai, T.F., Matsuura, T., Benton, C.S., Sutcliffe, J.S., Christian, S.L., Kubota, T., Halley, D.J., Meijers-Heijboer, H., et al (1999) The spectrum of mutations in UBE3A causing Angelman syndrome. Hum. Mol. Genet. 8, 129-135
34 Nawaz, Z., Lonard, D.M., Smith, C.L., Lev-Lehman, E., Tsai, S.Y., Tsai, M.J., O'Malley, B.W. (1999) The Angelman syndrome-associated protein, E6AP, is a coactivator for the nuclear hormone receptor superfamily. Mol. Cell. Biol. 19, 1182-1189
35 Smith, C.L., DeVera, D.G., Lamb, D.J., Nawaz, Z., Jiang, Y.H., Beaudet, A.L., O'Malley, B.W. (2002) Genetic ablation of the steroid receptor coactivator-ubiquitin ligase, E6AP, results in tissue-selective steroid hormone resistance and defects in reproduction. Mol. Cell. Biol. 22, 525-535
36 Jordan, C., Francke, U. (2006) Ube3a expression is not altered in Mecp2 mutant mice. Hum. Mol. Genet. 15, 2210-2215
37 Samaco, R.C., Hogart, A., LaSalle, J.M. (2005) Epigenetic overlap in autism-spectrum neurodevelopmental disorders: MECP2 deficiency causes reduced expression of UBE3A and GABRB3. Hum. Mol. Genet. 14, 483-492
38 Jiang, Y.H., Armstrong, D., Albrecht, U., Atkins, C.M., Noebels, J.L., Eichele, G., Sweatt, J.D., Beaudet, A.L. (1998) Mutation of the Angelman ubiquitin ligase in mice causes increased cytoplasmic p53 and deficits of contextual learning and long-term potentiation. Neuron 21, 799-811
39 Miura, K., Kishino, T., Li, E., Webber, H., Dikkes, P., Holmes, G.L., Wagstaff, J. (2002) Neurobehavioral and electroencephalographic abnormalities in Ube3a maternal-deficient mice. Neurobiol. Dis. 9, 149-159
40 Colas, D., Wagstaff, J., Fort, P., Salvert, D., Sarda, N. (2005) Sleep disturbances in Ube3a maternal-deficient mice modeling Angelman syndrome. Neurobiol. Dis. 20, 471-478
41 Albrecht, U., Sutcliffe, J.S., Cattanach, B.M., Beechey. C.V., Armstrong, D., Eichele, G., Beaudet, A.L. (1997) Imprinted expression of the murine Angelman syndrome gene, Ube3a, in hippocampal and Purkinje neurons. Nat. Genet. 17, 75-78
42 Cheron, G., Servais, L., Wagstaff, J., Dan, B. (2005) Fast cerebellar oscillation associated with ataxia in a mouse model of Angelman syndrome. Neuroscience 130, 631-637
43 Cheron, G., Servais, L., Dan, B., Gall, D., Roussel, C., Schiffmann, S.N. (2005) Fast oscillation in the cerebellar cortex of calcium binding protein-deficient mice: a new sensorimotor arrest rhythm. Prog. Brain Res. 148, 165-80
44 Kumar, S., Talis, A.L., Howley, P.M. (1999) Identification of HHR23A as a substrate for E6-associated protein-mediated ubiquitination. J. Biol. Chem. 274, 18785-18792
45 Oda, H., Kumar, S., Howley, P.M. (1999) Regulation of the Src family tyrosine kinase Blk through E6AP-mediated ubiquitination. Proc. Natl. Acad. Sci. USA 96, 9557-9562
46 Thomas, M., Banks, L. (1998) Inhibition of Bak-induced apoptosis by HPV-18 E6. Oncogene 17, 2943-2954
47 Kuhne, C., Banks, L. (1998) E3-ubiquitin ligase/E6AP links multicopy maintenance protein 7 to the ubiquitination pathway by a novel motif, the L2G box. J. Biol. Chem. 273, 34302-34309
48 Mani, A., Oh, A.S., Bowden, E.T., Lahusen, T., Lorick, K.L., Weissman, A.M., Schlegel, R., Wellstein, A., Riegel, A.T. (2006) E6AP mediates regulated proteasomal degradation of the nuclear receptor coactivator Amplified in Breast Cancer 1 in immortalized cells. Cancer Res. 66, 8680-8686
49 Ong, S.E., Blagoev, B., Kratchmarova, I., Kristensen, D.B., Steen, H., Pandey, A., Mann, M. (2002) Stable isotope labeling by amino acids in cell culture, SILAC, as a simple and accurate approach to expression proteomics. Mol. Cell. Proteomics 1, 376-86
50 Silva, J.C., Denny, R., Dorschel, C.A., Gorenstein, M., Kass, I.J., Li, G.Z., McKenna, T., Nold, M.J., Richardson, K., Young, P., Geromanos, S. (2005) Quantitative Proteomic Analysis by Accurate Mass Retention Time Pairs. Anal. Chem. 77, 2187-2200
51 Butz, K., Denk, C., Ullmann, A., Scheffner, M., Hoppe-Seyler, F. (2000) Induction of apoptosis in human papillomaviruspositive cancer cells by peptide aptamers targeting the viral E6 oncoprotein. Proc. Natl. Acad. Sci. USA 97, 6693-6697

### Background of the invention

Posttranslational modification of proteins by ubiquitin and ubiquitin-like proteins plays a prominent role in the control of many, if not all, cellular processes. In recent years, components of the respective conjugation systems have emerged as potential targets in the treatment of human diseases since they control pathways that for example, are of fundamental importance for the proliferative potential of cancerous cells or since their deregulation has been etiologically associated with the development of distinct disorders. An impressive example for the latter is represented by the E3 ubiquitin-protein ligase E6AP which is encoded by the *UBE3A* gene on chromosome 15q11-13 and has been associated with three distinct disorders. E6AP was originally isolated as an interacting protein of the E6 protein of oncogenic human papillomaviruses (HPV). In complex with E6, E6AP targets proteins for ubiquitination and degradation (e.g., the tumor suppressor p53) that are normally not recognized by E6AP, thereby contributing to HPV-induced cervical carcinogenesis. Inactivation of the E3 activity of E6AP by genetic means results in the development of the Angelman syndrome (AS), a neurodevelopmental disorder. Finally, deregulation of E6AP expression has been associated with autism spectrum disorders, and recent evidence obtained with transgenic mice inidcates that amplification of the *Ube3a* gene resulting in increased E6AP levels contributes to autistic phenotypes. Thus, both decreases and increases in E6AP activity contribute to the development of severe human pathologic conditions suggesting that E6AP activity has to be tightly controlled.

In general, E3 proteins are considered to mediate the specific recognition of substrates of the ubiquitin-conjugation system. Thus, identification and characterization of substrate proteins of E6AP should provide valuable insights into cellular processes/pathways that are controlled by E6AP and whose deregulation contributes to the different pathologic conditions. Several potential substrates of E6AP have been reported, including HHR23A and HHR23B, AIB1, PML, alpha-Synuclein, and Ring1B. However, the relevance of these interactions for AS development remains unclear. *Ube3a* knockout mice represent a good model for studying pathophysiological aspects of AS, as in many aspects, the phenotype of such mice resembles that of AS patients. Thus, to identify disease-relevant substrates of E6AP, Greer et al. (2010) crossed wild-type mice or *Ube3a* knockout mice with transgenic mice expressing HA-tagged ubiquitin. Proteins modified by HA-ubiquitin were isolated from brain lysates of the respective mice and compared by quantitative mass spectrometry. This resulted in the identification of Sacsin as a protein whose ubiquitination status is altered in the absence of E6AP. Sacsin contains a region with similarity to the XPC domain of the HHR23 proteins. As according to Greer et al. (2010) the synaptic protein Arc also contains an XPC-like domain, the authors hypothesized that XPC or XPC-like domains represent binding modules for E6AP and that Arc represents a ubiquitination substrate for E6AP. Indeed, data were provided to support this hypothesis followed by a thorough analysis of the role of E6AP in Arc-mediated endocytosis of AMPA receptors at excitatory synapses.

The neurophysiological data by Greer et al. (2010) clearly indicate that E6AP is involved in controlling the expression levels of Arc. However, in an effort to prove that XPC domains represent binding modules for E6AP, we found that E6AP binds to the ubiquitin-like (UBL) domains of the HHR23 proteins and of Sacsin rather than to the respective XPC domains. Similarly and posing a serious challenge to the model that Arc represents a direct ubiquitination substrate for E6AP, we provide evidence that E6AP does neither bind to the proposed XPC domain of Arc nor to full-length Arc and most importantly that E6AP does not target Arc for ubiquitination and degradation. Finally, we provide evidence for an alternative mechanism by which E6AP is involved in the control of Arc expression levels.

### Aim of the invention

Based on a novel role of the ubiquitin-ligase E6AP as modulator of steroid hormone receptor signalling it is an aim of the invention to provide a composition for use in the treatment of Angelman syndrome and/or autism spectrum disorder and methods for detecting mechanistic pathways thereof. Furthermore, the use of such composition and a method for manufacturing a medicament intended for therapeutic application in the treatment of Angelman syndrome and/or autism and/or autism spectrum disorders is provided.

### SUMMARY

The aforementioned aim of the invention is solved by a composition for use in the treatment of Angelman syndrome and/or autism and/or autism spectrum disorders, in accordance with the features of claim 1. Particularly, the composition comprises a substance that restores deregulated steroid hormone signaling and /or modulates the steroid hormone signaling in order to compensate for deregulation.

Further intended use of the composition is in the treatment of Epilepsy, Huntington's Disease, Prader-Willi syndrome, Cervical cancer, fragile X Syndrome, and/or Rett Syndrome.

An embodiment of the invention provides for a composition, wherein the substance is selected from the group consisting of steroid hormones and/or derivatives or functional analogues thereof, including androgens, glucocorticoids, progesterons, estrogenes, such as estradiols or derivates thereof, such as tamoxifen.

The aforementioned aim of the invention is further solved by use of a composition comprising a substance that restores deregulated steroid hormone signaling and /or modulates the steroid hormone signaling in order to compensate for deregulation for use in the treatment of Angelman syndrome and/or autism and/or autism spectrum disorders, or that restores and/or compensates the neuronal activity of E3 ubiquitin-protein ligase E6AP for use in the treatment of Angelman syndrome and/or autism and/or autism spectrum disorders. A further intended use of the composition is in the treatment of Epilepsy, Huntington's Disease, Prader-Willi syndrome, Cervical cancer, fragile X Syndrome, and/or Rett Syndrome.

An embodiment of the invention provides for a use of a composition comprising asubstance, wherein the substance is selected from the group of hormones, consisting of steroid hormones and/or derivatives thereof or functional analogues, including androgens, glucocorticoids, progesterons, estrogenes, such as estradiols or derivates thereof, such as tamoxifen.

The aforementioned object of the invention is further solved by a method for manufacturing a medicament intended for therapeutic application in the treatment of Angelman syndrome and/or autism and/or autism spectrum disorders, wherein a substance is used that increases and/or restores the expression levels of E3 ubiquitin-protein ligase E6AP.

A further embodiment of the invention provides for said method, wherein the substance is selected from the group consisting of estrogenes, particularly estrad iols.

Inactivation of the ubiquitin-protein ligase UBE3A/E6AP has been associated with development of the Angelman syndrome. Recently, it was reported that the synaptic protein Arc is targeted by E6AP for ubiquitination and degradation, providing a reasonable explanation for some phenotypic features of Angelman syndrome patients. Furthermore, it was proposed that similar to the known E6AP-interacting protein HHR23A, Arc contains an XPC domain and that XPC domains represent general E6AP-binding modules. In contrast to these previous results, we report here that E6AP binds to the UBL domain of HHR23A, and not to the XPC domain, and that E6AP does neither bind to Arc nor target it for ubiquitination and degradation. Instead, we provide evidence to suggest that E6AP is involved in controlling Arc levels by affecting nuclear hormone receptor-mediated transcription of the Arc gene.
□ E6AP binds to the UBL domain of HHR23A and Sacsin but not to their XPC domains
□ E6AP does not bind to Arc, which may contain an XPC domain but not a UBL domain
□ Arc is ubiquitinated within cells but this is not mediated by E6AP
□ Knockdown of E6AP expression stimulates estradiol-induced expression of the human Arc gene

### Results

### E6AP binds to the UBL domains of HHR23A and Sacsin

HHR23A and HHR23B belong to the family of ubiquitin-like (UBL) and ubiquitin-associated (UBA) domain-containing proteins that act as bridging factors between ubiquitinated proteins and the 26S proteasome (as the UBL, UBA, XPC domains of HHR23A and HHR23B are highly similar with more than 75 percent identity, we limited our analysis to HHR23A) (refs). In addition, it was reported that HHR23A represents a substrate for E6AP (ref). To delineate the region(s) of HHR23A that serve(s) as binding site(s) for E6AP, we expressed full-length HHR23A and several deletion mutants (Figure 1A) as GST fusion proteins in *E. coli* and tested their ability to bind to recombinant E6AP in coprecipitation experiments. This revealed that the N-terminal UBL domain is necessary and sufficient for E6AP binding (Figure 1 B).

In 2010, Greer et al. reported that an HHR23A mutant devoid of the XPC domain cannot interact with E6AP. Furthermore, based on sequence similarity searches, Greer et al. identified a region in Arc with limited similarity to the XPC domain of HHR23A and reported that an N-terminal truncation mutant of Arc binds to E6AP *in vitro* and that this interaction depends on the presence of the XPC domain of Arc. As these results were contradictory to our data, we revisited the interaction of HHR23A and E6AP. However, in our hands, an HHR23A mutant devoid of the XPC domain bound to E6AP with an efficiency similar to full-length HHR23A (Figure 1B, right panel), while we were not able to observe an interaction of E6AP with the isolated XPC domain (Figure 1C, right panel). Similarly, we were not able to obtain evidence for an interaction between E6AP and a GST fusion protein of full-length Arc or the proposed XPC domain of Arc (Figure 1C, left and right panel, respectively) (note that like Greer et al. (2010), we used mouse Arc rather than human ARC; the two proteins are 93 percent identical at the amino acid sequence level and, thus, can be considered as equivalent).

Since the XPC domains of HHR23A and Arc did not bind to E6AP, we finally extended our interaction studies to Sacsin which originally provided the link for Greer et al. (2010) to study the interaction of E6AP with Arc (as Sacsin contains an XPC domain (ref)). Again, we were not able to detect binding of the isolated XPC domain of Sacsin to E6AP (Figure 1 D, left panel). However, in addition to the XPC domain, Sacsin contains an N-terminal UBL domain (as indicated in the UniProtKB/Swiss-Prot database entry Q9NZJ4; Figure 1A), and indeed, similar to the results obtained for HHR23A, the UBL domain of Sacsin was able to bind to E6AP (Figure 1 D). Taken together, the data clearly show that XPC domains do not represent general binding modules for E6AP and that the UBL domain of Sacsin is sufficient for binding to E6AP (note that in contrast to HHR23A, we cannot conclude that the UBL domain is necessary for Sacsin to bind E6AP; Sacsin is a giant protein consisting of 4579 amino acids and, thus, additional regions of Sacsin may be able to mediate E6AP binding).

### Arc does not represent a ubiquitination or degradation substrate for E6AP within cells

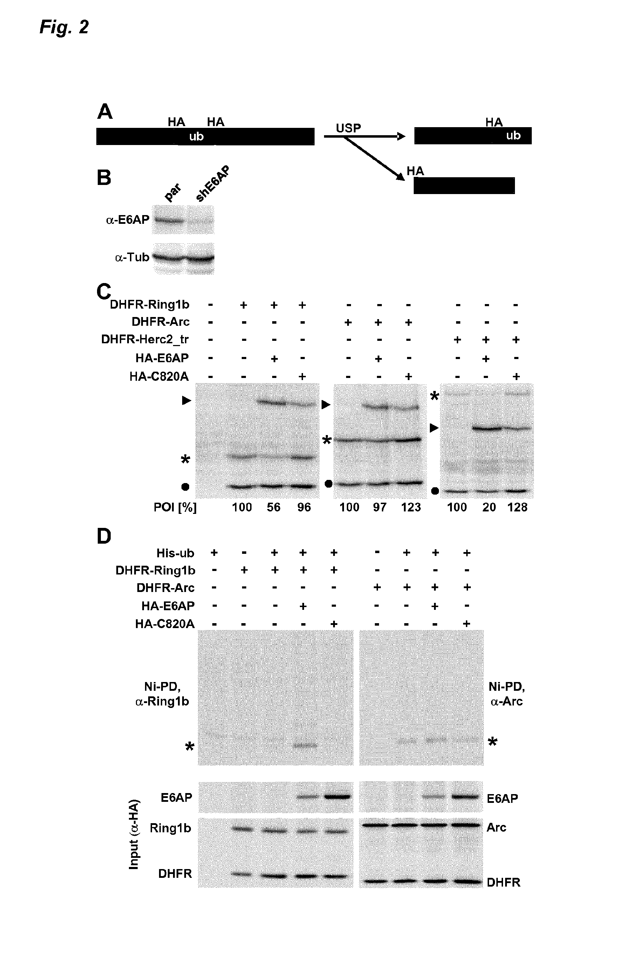

To provide evidence that E6AP can target Arc for ubiquitination and degradation within cells, Greer et al. (2010) determined the effect of ectopically expressed E6AP on ectopically expressed Arc in HEK293T cells in cotransfection experiments. Therefore, we performed similar experiments but rather than using conventional expression systems, we employed the DHFR-ubiquitin fusion protein system which is ideally suited for such studies (refs). This system takes advantage of the fact that ubiquitin-fusion proteins are cotranslationally cleaved by ubiquitin-specific proteases in a quantitative manner resulting in two separate proteins (Figure 2A). In brief, the cDNA encoding the protein of interest (POI) is fused in frame with a cDNA encoding a DHFR-ubiquitin fusion protein resulting in a construct expressing a single mRNA encoding a DHFR-ubiquitin-POI fusion protein. Since upon translation and concomitant cleavage, two separate proteins are generated from a common precursor protein (Figure 2A), comparison of the relative levels of the POI and DHFR-ubiquitin provides a direct measure to determine the effect of an E3 ligase on the ubiquitination and expression levels of a POI (note that a mutant form of ubiquitin, in which lysine residue 48 is replaced by arginine, is used in this system to avoid ubiquitination and degradation of DHFR-ubiquitin).

To determine if Arc represents a substrate for E6AP, Arc was expressed as a DHFR-ubiquitin fusion protein in the absence or presence of wild-type E6AP or a catalytically inactive mutant of E6AP in HEK293T cells in which endogenous E6AP expression is knocked down by RNA interference (Figure 2B). As controls, Ring1bI53S (a catalytically inactive form of the E3 ligase Ring1b), which was previously reported to represent a substrate for E6AP (refs), and an N-terminally truncated form of HERC2, which represents an artificial substrate of E6AP (HERC2_tr; for further details, see legend to Figure 2), were used. This revealed that coexpression of E6AP had no significant effect on Arc expression levels (Figure 2C). In contrast, expression levels of Ring1b-I53S and HERC2_tr were significantly reduced in the presence of wild-type E6AP but not in the presence of the catalytically inactive E6AP mutant (Figure 2C). Similarly, coexpression of E6AP had no significant effect on the levels of ubiquitinated Arc, while it facilitated (mainly) mono-ubiquitination of Ring1b-I53S, as reported (ref) (Figure 2D). Thus, the data indicate that within cells, Ring1b-I53S and HERC2_tr, but not Arc, represent substrates for E6AP-mediated ubiquitination and degradation.

### E6AP-mediated ubiquitination in vitro is an unspecific process

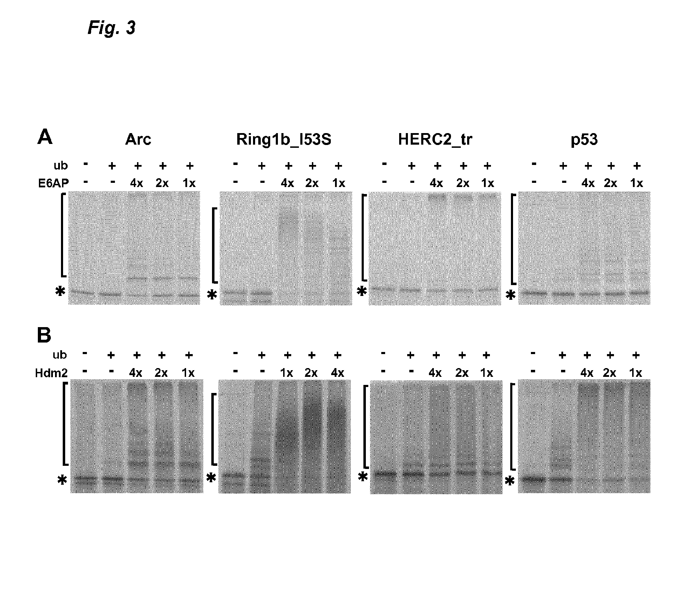

In support of the notion that Arc represents a substrate for E6AP, Greer et al. (2010) reported that Arc is ubiquitinated by E6AP *in vitro.* Thus, we performed in vitro ubiquitination experiments by incubating *in vitro* translated, radiolabeled Arc with recombinant ubiquitin-activating enzyme E1 and the ubiquitin-conjugating enzymes UbcH5b and/or UbcH7 in the absence or presence of ubiquitin and E6AP (for further details, see Experimental procedures). In parallel, ubiquitination assays with Ring1bI53S (ref) and HERC2_tr (see above) as substrates were performed (refs). As shown in Figure 3A, all three proteins were ubiquitinated by E6AP in a dose-dependent manner, though with somewhat different efficiencies.

At first glance, the observations that E6AP is capable of targeting Arc for ubiquitination *in vitro* but not within cells may seem to be contradictory. However, this apparent paradox is readily explained by the notion that in contrast to cell-based assays, the substrate specificity of *in vitro* ubiquitination systems appears to be rather limited. Firstly, although E6AP was originally isolated as a protein that in complex with HPV E6 targets p53 for ubiquitination and degradation, E6AP alone (i.e. in the absence of E6) does not appear to play a direct role in p53 turnover within cells (refs). However, we previously reported that although p53 neither binds to E6AP in a detectable manner nor represents a substrate for E6AP within cells, it is ubiquitinated by E6AP *in vitro* (ref) (see also Fig. 3A). Secondly, similar to the results obtained with E6AP, Arc, Ring1b, HERC2_tr, and p53 are ubiquitinated in the presence of the human ortholog of Mdm2 (Hdm2), an E3 ligase that is not related to E6AP (Fig. 3B). However, with the exception of p53 (ref), none of the proteins studied is known to interact with Hdm2, and we have no evidence to indicate that these proteins serve as substrates for Hdm2 within cells (data not shown). Finally, all the proteins studied are either known to be degraded by the ubiquitin-proteasome system (p53, Arc, Ring1b) and/or are components of it (Ring1b, HERC2) (refs; refs für arc). Thus, it seems likely that these proteins contain amino acid sequences or regions that serve as ubiquitination signals and that *in vitro,* these signals are promiscuous insofar as they are recognized by E3 ligases which are not involved in ubiquitination of the respective proteins within cells.

### Knockdown of E6AP expression results in increased estradiol-induced transcription of the Arc gene

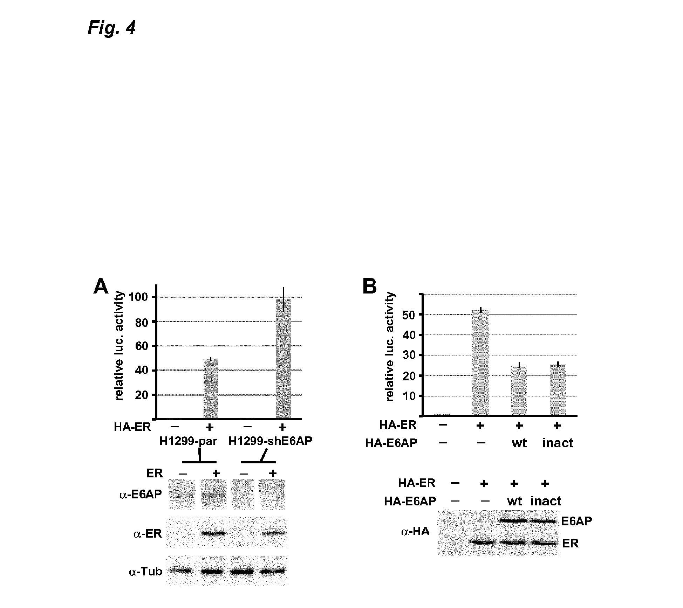

How can the data obtained so far be reconciled with the observation of Greer et al. (2010) that at least under certain conditions, Arc expression levels are increased in brain lysates derived from *Ube3a* null mice? E6AP was reported to affect nuclear hormone receptor-mediated transcription by E3 ligase-independent and E3 ligase-dependent mechanisms (refs). Furthermore, expression of the human *Arc* gene was reported to be influenced by estradiol signaling (refs). We, therefore, hypothesized that loss of E6AP may affect estrogen receptor (ER)-mediated transcription and thereby Arc expression. To address this possibility, we first studied ER-induced transcription of a respective reporter construct in H1299-shE6AP cells, in which E6AP expression is knocked down by RNA interference (ref), and in parental H1299 cells. Indeed, ER-induced expression of the reporter construct was approximately 2fold higher in H1299-shE6AP cells than in H1299 cells (Figure 4A). In contrast, ectopic expression of E6AP decreased ER-induced transcription by a factor of 2-3 and this repressive effect was not dependent on E3 ligase activity (Figure 4B).

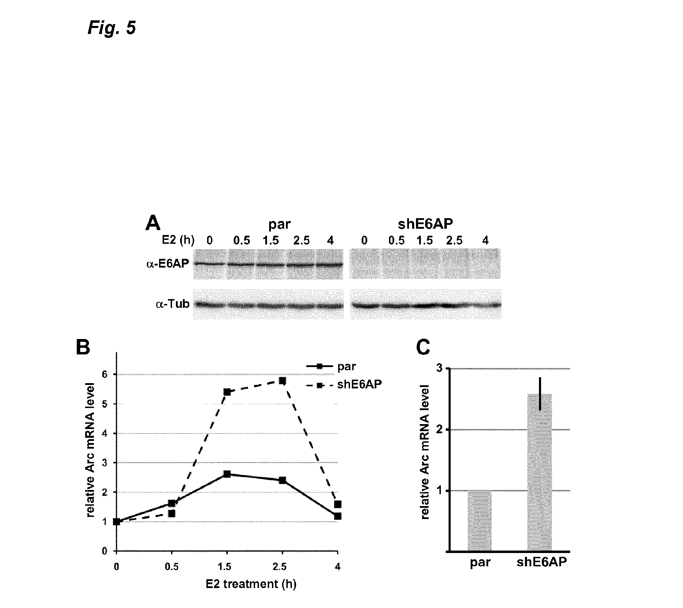

Having shown that loss of E6AP expression stimulates ER-induced transcription of a reporter gene, we then determined the effect of estradiol treatment on endogenous Arc mRNA levels in neuroblastoma-derived SH-SY5Y cells and in SH-SY5Y-shE6AP cells, in which E6AP expression is knocked down by RNA interference (Figure 5A). As reported (ref), treatment of parental SH-SY5Y cells with estradiol resulted in a transient increase of Arc mRNA levels (Figure 5B). In line with the results obtained with the ER-responsive reporter construct, this increase was 2-3fold higher in SH-SY5Y-shE6AP cells (Figures 5B and 5C).

In conclusion, our data indicate that E6AP can function as a repressor of estradiol-induced transcription and that at least under certain conditions, *Arc* gene expression is controlled by E6AP.

### DISCUSSION

All of the genetic abnormalities associated with development of AS, including deletion of the 15q11-13 region of the maternal chromosome, uniparental paternal disomy, and single point mutations in the *UBE3A* gene, result in loss of E6AP expression or expression of mutated forms of E6AP (refs). Notably, the single point mutations studied so far result in E6AP mutants with reduced E3 ligase activity (refs) indicating that constitutive or transient increases in the expression level of substrate proteins of E6AP play a major role in AS development. Thus, identification of E6AP substrates may not only provide insight into the processes and pathways underlying AS but may also reveal potential targets for therapeutic strategies. Arc is commonly accepted as a crucial player in synaptic plasticity, deregulation of which contributes to the manifestation of AS (refs). Thus, the finding of Greer et al. (2010) that at least under certain circumstances, Arc expression levels are elevated in the absence of E6AP is likely to explain some of the symptoms of AS patients. While our data support the notion that loss of E6AP expression results in deregulation of Arc, we disagree with Greer et al. (2010) over the mechanism underlying this phenomenon. In contrast to Greer et al. (2010), we found that Arc does not represent a proteolytic substrate for E6AP and that E6AP affects Arc expression on the transcriptional rather than on the posttranslational level.

When considering possible causes for the discrepancies between our data and those of Greer et al. (2010), one should recollect that Greer et al. rather indirectly arrived at studying the possibility that Arc represents a substrate for E6AP. As aforementioned (see Introduction), they isolated Sacsin from mouse brain lysates as a protein, whose ubiquitination status differs in the absence and in the presence of E6AP. Since similar to HHR23A (a known E6AP-interacting protein) Sacsin contains an XPC domain (refs), Greer et al. then proposed that XPC or XPC-like domains might serve as E6AP binding domains. In support of their hypothesis, Greer et al. provided data that E6AP binds *in vitro* to full-length HHR23A but not, or less efficiently, to a mutant of HHR23A lacking the XPC domain. However, no evidence was provided that the proposed XPC domain of Sacsin binds to E6AP or that the XPC domain of HHR23A is sufficient for E6AP binding. In this study, we found that the XPC domain of neither HHR23A nor Sacsin binds to E6AP in a detectable manner. Moreover, both HHR23A and Sacsin contain an N-terminal UBL domain, and the isolated UBL domain of either protein is sufficient to mediate binding to E6AP. Furthermore, in our hands the HHR23A mutant lacking the XPC domain binds to E6AP as efficient as full-length HHR23A. We cannot explain the different results obtained by Greer et al. and by us with respect to the HHR23A deletion mutant. However, our data clearly indicate that the XPC domains of HHR23A and Sacsin do not represent a binding site for E6AP (Figure 1).

Based on the rationale that XPC domains represent E6AP binding domains, Greer et al. (2010) also identified a region of Arc with rather limited similarity to the XPC domain of HHR23A. Again, we were not able to detect an interaction between E6AP and the proposed XPC domain of Arc (this was not tested by Greer et al.) and did also not observe an interaction between E6AP and full-length Arc *in vitro* (Figure 1 C) or within cells in overexpression experiments (not shown). The reasons for the difference in the results obtained for the interaction between E6AP and full-length Arc remain unclear. One possibility could be that E6AP binds to Arc with rather low affinity and, therefore, detection of this interaction may strongly depend on the conditions used. However, we were not able to observe a specific interaction of E6AP with Arc under any condition tested (e.g., increasing the amounts of both proteins, decreasing salt concentration). On the other hand, the results obtained in *in vitro* ubiquitination assays by us (Figure 3) and by Greer et al. (2010) indicate that E6AP has the ability to ubiquitinate Arc. However, as discussed above (see Results), we propose that this ubiquitination of Arc is not specific (note that Greer et al. did not provide controls to indicate that the ubiquitination observed *in vitro* is specific). Arc is degraded via the ubiquitin-proteasome system (refs). Thus, by definition it contains regions/amino acid sequences that serve as ubiquitination signal, and this signal apparently suffices to be recognized by different E3 ligases (E6AP, Hdm2) *in vitro,* but not in a cellular environment. In other words, *in vitro* ubiquitination studies are important tools to provide support for the notion that a protein is a direct substrate for a given E3 ligase, but additional criteria need to be met to conclude that the ubiquitination observed depends on a specific interaction.

An important cornerstone in the line of arguments that a certain E3 ligase mediates ubiquitination and degradation of a given protein is the verification that the E3 ligase affects the turnover rate and the ubiquitination status of the respective protein in coexpression experiments within cells. Similar to our data (Figure 2D), Greer et al. observed ubiquitination of Arc in the absence of ectopically expressed E6AP. In contrast to our data, however, coexpression of E6AP resulted in somewhat increased levels of ubiquitinated Arc, while ubiquitinated Arc species were not observed in the presence of a catalytically inactive E6AP mutant. A potential drawback in cotransfection experiments is that transfection efficiencies can vary between different transfections and, thus, transfection efficiencies need to be taken into account when interpreting results obtained in such experiments (we do not know if this was done by Greer et al.). To do so, the DHFR-ubiquitin fusion protein system (ref) is ideally suited, since the relative transfection efficiency can be readily determined by monitoring the level of DHFR-ubiquitin (Figure 2A). Using this system, we did not obtain evidence that E6AP has a significant effect on the ubiquitination status of ectopically expressed Arc. Similarly, we could not confirm the results of Greer et al. that coexpression of E6AP results in decreased Arc levels (Figure 2C). In this context, it should be noted that although Greer et al. observed a significant decrease in Arc levels upon coexpression of E6AP in the experiment shown, they did not perform (or at least did not show) proteasome inhibitor experiments or half-life experiments to provide direct evidence that the decrease is indeed due to enhanced degradation rather than to effects on, for instance, the transcriptional or translational level. In contrast, such experiments are not required for the interpretation of data obtained in the DHFR-ubiquitin fusion protein system, since DHFR-ubiquitin and the protein of interest are expressed as a single polyprotein from the same mRNA and, thus, determination of the relative ratio between the protein of interest and DHFR-ubiquitin provides unequivocal evidence as to whether a protein is preferentially degraded or not.

Arc is a short-lived protein (refs) and Arc expression is also known to be regulated at the transcriptional level in response to certain stimuli (e.g., refs.). Greer et al. (2010) showed that upon treatment of hippocampal cells with kainate, Arc protein levels but not Arc mRNA levels are increased in E6AP-deficient cells. At first glance, an obvious explanation for these observations is that the half-life of the Arc protein is increased in the absence of E6AP. However, Arc transcription is rapidly but also only transiently induced in response to various stimuli (refs). For instance, similar to our results (Figure 5), it was reported that treatment of SH-SY5Y cells with estradiol or carbachol results in a rapid but transient induction of Arc mRNA levels and that this transient increase in mRNA levels is mirrored in a transient increase in Arc protein levels but with different kinetics (refs). Since Greer et al. performed their analysis at a single time point rather than in a time course experiment, we propose that the increase of Arc protein levels observed in E6AP null cells was due to a transient increase in mRNA levels rather than a prolonged half-life of the Arc protein.

It was recently reported that transgenic mice carrying a third *Ube3a* allele show autism-like features (ref). Of note, all three Ube3a alleles were engineered in a manner that they express E6AP with an additional C-terminal FLAG epitope. To provide evidence that "E6AP-FLAG" is still active as an E3 ligase, it was shown that in these mice, Arc expression levels are significantly decreased in brain-derived lysates. However, it was previously reported that fusing a C-terminal extension (e.g., FLAG epitope) to E6AP as well as the HECT E3 ligase Rsp5 results in a ubiquitination-defective protein (ref). We, therefore, generated an E6AP variant with a C-terminal FLAG epitope and, as reported, this E6AP variant is severely compromised in its E3 activity (Supplemental Figure 2). Furthermore, ectopic expression of a catalytically inactive E6AP mutant decreased ER-induced transcription of a reporter construct with an efficiency similar to wild-type E6AP (Figure 5B) indicating that at least in the system used, the E3 activity of E6AP is not essential for its transcriptional effect. Combining these results, we propose that the decrease in Arc levels observed by Smith et al. (2011) is not due to increased E6AP-faclitated degradation of Arc but rather to deregulated transcription of the *Arc* gene (or in other words, determining Arc protein levels is not a suitable means to score for E3 ligase activity of E6AP).

Besides posing a serious challenge to the previously proposed mechanism by which E6AP regulates Arc protein levels, our data indicate that the importance of the connection between E6AP and nuclear hormone receptors for AS development may have been underestimated so far. Estrogen was reported to affect Arc transcription (ref) as well as various neuronal processes including synaptic plasticity (refs). Furthermore, approx. 5-10 percent of AS patients do not harbor a detectable defect in the *UBE3A* gene (ref). Thus, although we do not yet know the exact mechanism, by which E6AP affects estradiol-induced transcription of the *Arc* gene (as indicated in the Introduction, it was reported that E6AP can affect transcriptional processes by E3-dependent and E3-independent mechanisms (refs)), it is tempting to speculate that some of the AS patients may carry defects along the respective signaling pathway(s). Furthermore, analysis of transgenic mice, in which E6AP expression is ablated in mammary tissue, identified a few genes, whose estradiol-induced expression is down-regulated in the absence of E6AP (refs). Similarly, we have preliminary data to suggest that in contrast to the *Arc* gene, downregulation of E6AP expression in SH-SY5Y results in a significant decrease in estradiol-induced transcription of the *CTSD* gene, an established estradiol-responsive gene encoding cathepsin D (ref) (Supplementary Figure 3). Taken together, these data support the notion that E6AP has both positive and negative effects on estradiol-mediated transcription. Thus, delineation of the mechanism(s) involved in this phenomenon and identification of the genes whose expression is up- or down-regulated in the absence of E6AP should contribute to the elucidation of the pathways involved in AS development.

### EXPERIMENTAL PROCEDURES

### Cell Lines and Plasmids

H1299 cells and HEK293T cells were grown in DMEM supplemented with 10% (v/v) FBS. SH-SY5Y cells were grown in DMEM/F12 (1:1) +GlutaMAX supplemented with 10% (v/v) FBS.

The bacterial expression constructs for the GST fusion proteins of full-length HHR23A, HHR23B, and Arc and of various deletion mutants of HHR23A, Arc, and Sacsin (Figs. 1 and 2) were generated by PCR-based approaches (further details will be provided upon request). Expression constructs (*in vitro* translation, transient transfection experiments) encoding HA-tagged wild-type E6AP (isoform 1), the HA-tagged catalytically inactive mutant E6AP-C820A (substitution of Cys-820 by Ala), the catalytically inactive mutant Ring1B-I53S, human p53, and His-tagged ubiquitin were described previously (refs). Expression constructs (transient transfection experiments) encoding HA-tagged Arc, HA-tagged HERC_tr (amino acids 2958-4834 of HERC2), and HA-tagged ER-α as well as DHFR-HA-ubiquitin fusion proteins (ref) of HA-Arc, HA-Ring1B-I53S, and HA-HERC2_tr were generated by PCR-based approaches (further details will be provided upon request). The estrogen receptor-responsive reporter construct (3xERE TATA Luc) was obtained from Addgene (Hall and McDonnell, 1999).

### Transfection and Antibodies

For transient expression, cells were transfected with the respective constructs by lipofection (Lipofectamine 2000; Invitrogen) according to the manufacturer's instructions. Protein extracts were prepared 24 h after transfection as described (ref). Levels of the various HA-tagged proteins were determined by Western blot analysis using the mouse monoclonal HA.11 (Hiss Diagnostics, Freiburg, Germany). Where indicated, Arc, Ring1B-I53S, E6AP, and Tubulin were detected by an anti-Arc mouse monoclonal antibody (BD Transduction Laboratories, 612602), an anti-Ring1b antibody (MBL International), a mouse monoclonal antibody against E6AP (ref), and an anti-Tubulin mouse monoclonal antibody (Abcam).

To generate cell lines, in which E6AP expression is stably suppressed by RNA interference, HEK293T cells were transfected with pMSCV-Hygro-NA8 (Clontech) and SH-SY5Y cells were transfected with pcDNA4TO-NA8-IRES-Puro by lipofection (Lipofectamine 2000, Invitrogen) and nucleofection (Amaxa® Cell Line Nucleofector® Kit V), respectively. Both constructs express an shRNA directed against nucleotides 300-318 of the E6AP mRNA (numbering according to E6AP isoform 1, with nucleotide 1 referring to A of the start codon (ref)). Cells stably containing the respective expression construct were selected by resistance to hygromycin (SIGMA) and puromycin (SIGMA), respectively. After establishing respective cell lines, protein extracts were prepared, and E6AP levels were determined by Western blot using an anti-E6AP mouse monoclonal antibody (ref).

### Coprecipitation, Ubiquitination, and Degradation Assays

*In vitro* coprecipitation experiments using GST fusion proteins were performed as described previously (ref). Briefly, 500 ng of baculovirus expressed E6AP were incubated with 1 µg of bacterially expressed GST or GST fusion proteins as indicated (Figs. 1 and 2). After 4 h, bound proteins were purified by glutathione affinity chromatography and electrophoresed in 8% SDS-polyacrylamide gels, and E6AP was detected by Western blot analysis.

For *in vitro* ubiquitination experiments, the ubiquitin-activating enzyme E1 and E6AP (isoform 1) were expressed in the baculovirus system, and UbcH5b and UbcH7 were expressed in *E*. *coli* BL21 by using the pET expression system as described (ref). For *in vitro* ubiquitination, 1µl of rabbit reticulocyte lysate-translated ³⁵S_labeled substrate (Arc, HERC2_tr, p53, Ring1b-I53S) was incubated with 50 ng of E1, 50 ng of UbcH5b or UbcH7, increasing amounts (100-400 ng) of baculovirus-expressed E6AP or Mdm2, and 20 µg of ubiquitin (Sigma) in 40µl volumes. In addition, reactions contained 25 mM Tris-HCl (pH7.5), 50 mM NaCl, 1 mM DTT, 2 mM ATP, and 4 mM MgCl₂. After incubation at 25°C (E6AP) or 35°C (Mdm2) for 2 h, total reaction mixtures were electrophoresed in 10% SDS-poly- acrylamide gels, and ³⁵S-labeled proteins were detected by fluorography.

For ubiquitination of Arc and Ring1B-I53S within cells, one 6-cm plate of HEK293T-K2 cells (stable knockdown of E6AP expression) was transfected with expression constructs encoding E6AP or the catalytically inactive mutant E6AP-C820A (2.5 µg), His-tagged ubiquitin (1.5 µg), and DHFR-HA-ubiquitin-HA-Ring1BI53S (1 µg) or DHFR-HA-ubiquitin-HA-Arc (1 µg) as indicated (Figure 3B). 24 h after transfection, 30% of the cells were lysed under non-denaturing conditions as described (ref) to determine expression levels of the two forms of E6AP, DHFR-HA-ubiquitin, HA-Ring1B-I53S, and HA-Arc. The remaining cells were lysed under denaturing conditions, and ubiquitinated proteins were purified as described (ref). Degradation assays were performed similar to ubiquitination assays, except that 3.5 µg and 1 µg of the expression constructs encoding E6AP and E6AP-C820A, respectively, were used and the expression construct for His-ubiquitin was omitted. Quantification of the intensity of the signals was performed with the Aida 4.08 software package (Raytest, Germany).

### Luciferase reporter assays

Transfections of H1299 or H1299-shE6AP (stable knockdown of E6AP expression (ref)) cells were performed in 6-well plates. Per well, 1 µg of the ER reporter construct, 400 ng of a β-galactosidase expression construct, and 50 ng of the ERα expression construct were transfected as indicated (Figure 4A) Brief description of conditions for Fig. 4B. 24 h after transfection, cells were harvested and lysed on ice in 120 µl 1 x Luciferase Cell-Culture Lysis Reagent (Promega). After 30 min, samples were centrifuged for 15 min at 4°C and the supernatant used to determine both luciferase activity and β-galactosidase activity to adjust for transfection efficiency. Furthermore, each transfection was performed in duplicate. Luciferase assays were performed using a Luciferase Assay System kit (Promega) and analyzed on a Wallac 1420 multilabel counter (PerkinElmer).

### Estradiol treatment and Real Time PCR

Parental SH-SY5Y cells and SH-SY5Y-shE6AP cells were starved overnight in phenol-red-free medium containing 5% charcoal-stripped FBS. Medium was exchanged and cells were treated with 10 nM 17β-Estradiol (Sigma). Approx. 5x 10⁶ cells were harvested at the times indicated (Figure 5) and RNA isolated by using TRlzol® Reagent (Invitrogen) and a Genejet RNA purification column (Fermentas) according to the manufacturers' instructions. Reverse transcription was performed using the SuperScript® III Reverse Transcriptase Kit (Invitrogen) according to the manufacturer's instructions. The resulting cDNA was purified using the GeneJet PCR purification kit (Fermentas).

Quantitative PCR analysis was performed using the MaximaTM SYBR Green/ROX qPCR Master Mix (Fermentas) or FastStart Universal SYBR Green Master Mix (ROX) (Roche) on the StepOne Quantitative PCR machine (Applichem). The Primers used have the following sequences: Arc forward primer, CGCGAGGTGTTCTAC, Arc reverse primer, AGCCAGTACTCCTCAG; Actin forward primer, GCTCCGGCATGTGCAA, Actin reverse primer, TGGCACCACACCTTCTAC. mRNA expression levels were compared using the ΔΔ-Ct method (ref). Actin mRNA was chosen as reference target.

### FIGURE LEGENDS

**Figure 1****. E6AP does not bind to XPC domains but to the UBL domain of HHR23A and Sacsin**
   (A) Schematic structure of HHR23A, HHR23A deletion mutants, Sacsin, and Arc. The position of ubiquitin-like domains (UBL), ubiquitin-associated domains (UBA1, UBA2), and XPC domains are indicated.
   (B-D) HHR23A and its various deletion mutants, Arc and its proposed XPC domain (Arc_XPC; amino acid residues 255-318 (Greer et al., 2010)), and the XPC domain and the UBL domain of Sacsin (SAC_XPC, SAC_UBL) were bacterially expressed as GST fusion proteins and purified by affinity chromatography using glutathione-Sepharose (lower panel). Upon adjustment of protein amounts (see Suppl. Figure 1), the various fusion proteins were incubated with baculovirus-expressed E6AP. After 4 h at 4°C, glutathione beads were centrifuged, supernatants were removed, and beads were washed. The amount of E6AP bound to the various proteins was determined by SDS-PAGE followed by Western blot analysis. Input, corresponds to 20% of E6AP used for binding reactions.
   Note that similar results (i.e. Arc does not bind to E6AP) were obtained with a bacterially expressed GST fusion protein of E6AP and *in vitro* in rabbit reticulocyte lysate translated radiolabeled Arc (not shown) and that UBL domains do not generally represent E6AP binding domains, since UBL domains of other proteins (e.g., hPLIC-2) do not detectably bind to E6AP (not shown).
**Figure 2****. Arc does not represent a substrate for E6AP in cells**
   (A) Schematic of the DHFR-HA-ubiquitin fusion protein system. The cDNA encoding the protein of interest (POI) is fused in frame with a cDNA encoding a DHFR-HA-ubiquitin fusion protein resulting in a construct expressing a single mRNA encoding a DHFR-HA-ubiquitin-HA-POI fusion protein. Such fusion proteins are cotranslationally cleaved by ubiquitin-specific proteases (USP) in a quantitative manner so that, within cells, two separate proteins (DHFR-HA-ub and HA-POI) are generated by a single mRNA.
   (B) Extracts were prepared from HEK293T cells (par) and from HEK293T cells, in which endogenous E6AP expression was stably down-regulated by RNA interference (shE6AP), and subjected to Western blot analysis with antibodies directed against E6AP and tubulin (Tub) as indicated.
   (C) HEK293T-shE6AP cells were cotransfected with expression constructs for HA-tagged wild-type E6AP or its catalytically inactive mutant E6AP-C820A, and DHFR-HA-ubiquitin fusion proteins of HA-tagged Ring1b-I53S, HA-tagged Arc, or HA-tagged HERC2_tr as indicated. Protein extracts were prepared 24 h after transfection and levels of the various proteins were determined by Western blot analysis using an anti-HA antibody. The intensity of the various HA-tagged proteins was quantified. The relative ratio of HA-tagged Ring1b-I53S, Arc, or HERC2_tr to DHFR-HA-ubiquitin is indicated, respectively, with the ratio of HA-tagged Ring1b-I53S, Arc, or HERC2_tr to DHFR-HA-ubiquitin in the absence of ectopically expressed E6AP set to 100%. Running positions of DHFR-HA-ubiquitin are indicated by closed circles, and those of HA-tagged Ring1b-I53S, Arc or HERC2_tr are indicated by asterisks. Running positions of E6AP and its catalytically inactive mutant C820A are indicated by arrowheads.
   (D) Transfections were performed as in (C) but in presence of an expression construct for His-tagged ubiquitin (His-ub). 24 h after transfection, protein extracts were prepared and ubiquitinated proteins were isolated by Ni²⁺⁻affinity chromatography (Ni-PD). Upon purification, levels of ubiquitinated Ring1b-I53S and Arc were determined by Western blot analysis with antibodies directed against Ring1b or Arc as indicated. Input, corresponds to 15% of the protein extracts used for affinity purification. *, (presumably mono-)ubiquitinated Ring1b-I53S and Arc, respectively.
   Ring1b-I53S was used as substrate rather than wild-type Ring1b, because due to the mutation, Ring1b-I53S is severely compromised in its function as E3 ligase and cannot ubiquitinate itself. Thus, degradation (C) and ubiquitination (D) of Ring1b-I53S by E6AP can be easily monitored. HERC2_tr was used rather than full-length HERC2, because it still binds to E6AP (ref) but unlike full-length HERC2, it represents an efficient substrate for E6AP.
**Figure 3****. E6AP is a promiscuous ubiquitin-protein ligase *in vitro***
   *In vitro* translated radiolabeled Arc, p53, Ring1b-I53S, or an N-terminal truncation mutant of HERC (HERC2_tr comprising amino acid residues 2958-4834 of HERC2) were incubated in the absence or presence of ubiquitin (ub) with increasing amounts of baculovirus-expressed E6AP (A) or bacterially expressed GST fusion protein of Hdm2 (B) under standard ubiquitination conditions (Experimental procedures). After 120 min, reactions were stopped and reaction products analyzed by SDS-PAGE followed by fluorography. Running positions of the non-modified form and of the ubiquitinated forms of the various substrates are indicated by asterisks and brackets, respectively.
   Ring1b-I53S was used as substrate rather than wild-type Ring1b, because due to the mutation, Ring1b-I53S is severely compromised in its function as E3 ligase and cannot ubiquitinate itself. Thus, ubiquitination of Ring1b-I53S by other E3 ligases can be easily monitored. HERC2_tr was used, because unlike full-length HERC2, it represents an efficient substrate for E6AP.
**Figure 4****. E6AP expression levels affect estrogen receptor-mediated transcription**
   (A) H1299 cells (H1299-par) and H1299 cells, in which endogenous E6AP expression is stably down-regulated by RNA interference (H1299-shE6AP) (ref), were cotransfected with an estrogen receptor-responsive reporter construct encoding luciferase and an expression construct for β-galactosidase (to determine transfection efficiency) in the absence (-) or presence (+) of an expression construct encoding an HA-tagged??? form estrogen receptor-α (ER). Protein extracts were prepared 24 h after transfection. Luciferase activity was determined as measure for ER-α activity and the relative values obtained adjusted for transfection efficiency. 20% of the extracts were subjected to Western blot analysis with antibodies directed against E6AP, HA-???ER-α, and tubulin (Tub) as indicated.
   (B) Transfection of (parental) H1299 cells was performed as in (A) but in the presence of HA-tagged wild-type E6AP (wt) or a catalytically inactive mutant of E6AP (inact) as indicated. Analysis was performed as in (A).
**Figure 5****. Knockdown of E6AP expression levels stimulates estradiol-mediated transcription of the human Arc gene**
   SH-SY5Y cells (par) and SH-SY5Y cells, in which endogenous E6AP expression is stably down-regulated by RNA interference (shE6AP), were treated with 10 nM estradiol (E2) and cells were harvested at the times indicated. (A) 10% of the cells were used to determine the expression levels of E6AP (α-E6AP). Analysis of tubulin levels (α-Tub) served as loading control. (B) The remaining cells were used to isolate total RNA followed by cDNA synthesis and determination of relative levels of Arc mRNA by real time PCR. Simultaneous determination of Actin mRNA levels served as reference. The ratio between Arc mRNA and Actin mRNA at time 0 was set to 1. The Real time PCR experiment was performed four times with similar results but the time course of estradiol-induced transcription of the *Arc* gene varied slightly between individual experiments. The graph shows a representative example. (C) Summary of the data obtained in four different experiments. The maximum level of Arc mRNA observed upon addition of estradiol to parental cells (e.g., mRNA level at 1.5 h in Fig. 5B) was set to "1" for each individual experiment and the relative increase of Arc mRNA levels observed in E6AP knockdown cells (shE6AP) was correlated accordingly.

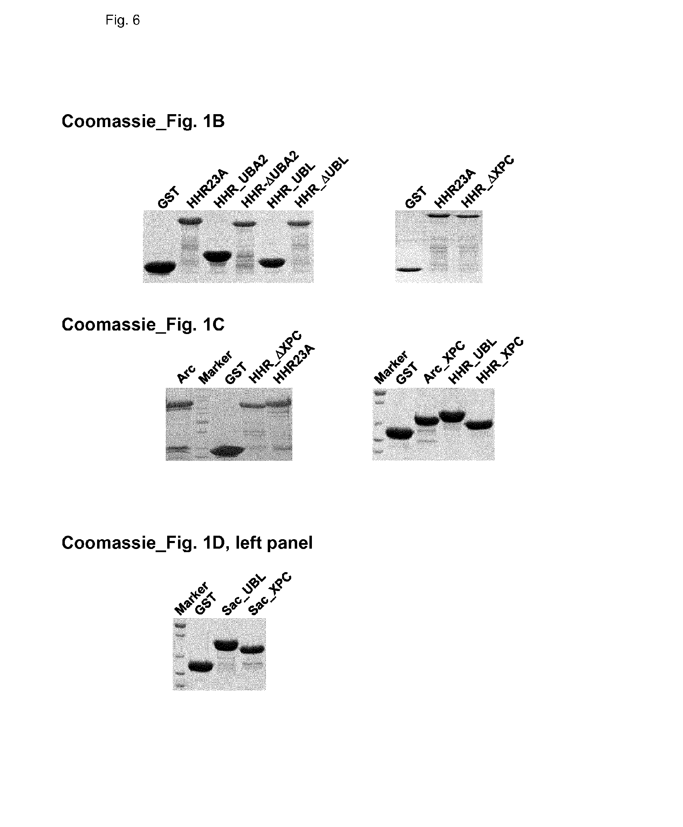

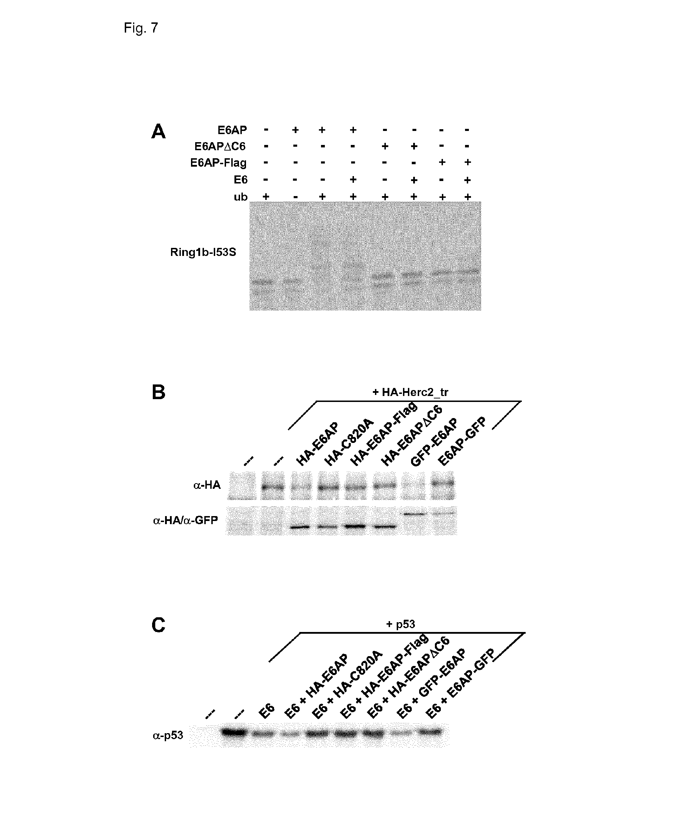

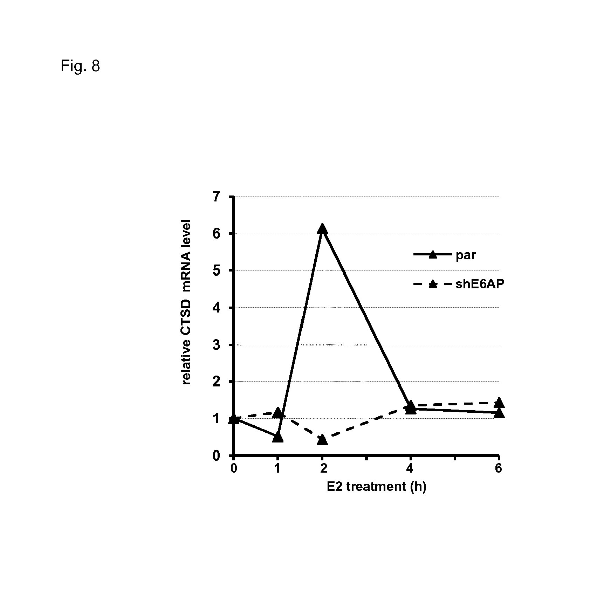

**Figures 6****,** **7****, and** **8** **are merely shown as supplementary drawings.**

## Claims

1. Composition for use in the treatment of Angelman syndrome and/or autism spectrum disorder, wherein the composition comprises a substance that restores deregulated steroid hormone signaling and /or restores the expression levels or neuronal activity of E3 ubiquitin-protein ligase E6APmodulates the steroid hormone signaling in order to compensate for deregulation

2. Composition according to claim 1, wherein the substance is selected from the group of hormones, consisting of steroid hormones and/or derivatives or functional analogues including androgens, glucocorticoids, progesterons, estrogenes, such as estradiols or derivates thereof, such as tamoxifen.

3. Use of a composition comprising a substance that restores deregulated steroid hormone signalling and /or modulates the steroid hormone signaling in order to compensate for deregulation for use in the treatment of Angelman syndrome and/or autism and/or autism spectrum disorders, or that increases and/or restores and/or compensates the expression levels or the neuronal activity of E3 ubiquitin-protein ligase E6AP for use in the treatment of Angelman syndrome and/or autism and/or autism spectrum disorders.

4. Use according to claim 3, wherein the substance is selected from the group of hormones, consisting of steroid hormones and/or derivatives thereof or functional analogues including androgens, glucocorticoids, progesterons, estrogenes, such as estradiols or derivates thereof, such as tamoxifen.

5. Method for manufacturing a medicament intended for therapeutic application in the treatment of Angelman syndrome and/or autism and/or autism spectrum disorders, or that increases and/or restores and/or compensates the expression levels or the neuronal activity of E3 ubiquitin-protein ligase E6AP for use in the treatment of Angelman syndrome and/or autism and/or autism spectrum disorders.

6. Method according to claim 5, wherein the substance is selected from the group of hormones, consisting of steroid hormones and/or derivatives thereof or functional analogues including androgens, glucocorticoids, progesterons, estrogenes, such as estradiols or derivates thereof, such as tamoxifen.
